Europäisches Patentamt

European Patent Office

Office européen des brevets

(19)

(11) Veröffentlichungsnummer: **0 275 880 B1**

# EUROPÄISCHE PATENTSCHRIFT

(12)

(45) Veröffentlichungstag der Patentschrift: 24.07.91

(51) Int. Cl.⁵: **A61K 31/12**, A61K 9/14,
A61K 47/12, A61K 47/04

(21) Anmeldenummer: 88100144.0

(22) Anmeldetag: 08.01.88

Die Akte enthält technische Angaben, die nach
dem Eingang der Anmeldung eingereicht wurden
und die nicht in dieser Patentschrift enthalten sind.

(54) Verfahren zur Herstellung von stabilisierten Menadionbisulfit-Formulierungen.

(30) Priorität: 14.01.87 DE 3700812

(43) Veröffentlichungstag der Anmeldung:
27.07.88 Patentblatt 88/30

(45) Bekanntmachung des Hinweises auf die
Patenterteilung:
24.07.91 Patentblatt 91/30

(84) Benannte Vertragsstaaten:
BE DE ES FR GB IT NL

(56) Entgegenhaltungen:
DE-A- 1 202 624

(73) Patentinhaber: BASF Aktiengesellschaft
Carl-Bosch-Strasse 38
W-6700 Ludwigshafen(DE)

(72) Erfinder: Kiefer, Hans, Dr.
Im Sandgarten 5
W-6706 Wachenheim(DE)
Erfinder: Buehler, Wolfgang, Dr.
Neckarstrasse 12
W-6832 Hockenheim(DE)
Erfinder: Schneider, Joachim U., Dr.
Plauserstrasse 17
W-6719 Weisenheim(DE)
Erfinder: Jaedicke, Hagen, Dr.
Anglerstrasse 38
W-6700 Ludwigshafen(DE)

EP 0 275 880 B1

## Beschreibung

Die Erfindung betrifft Verfahren zur Herstellung von stabilisierten Menadionbisulfit-Formulierungn. 2-Methyl-naphthochinon-1,4 (Menadion), das man zu den Vitaminen der K-Reihe zählt, besitzt bekanntlich eine hohe antihämorrhagische Wirkung. In der Literatur wird Menadion überwiegend als Vitamin $K_3$ bezeichnet (vgl. Ullmanns Encyklopädie der technischen Chemie, 4. Auflage, Band 23, S. 649 ff, Verlag Chemie, Weinheim, 1983).

Trotz der hervorragenden antihämorrhagischen Wirkung des Menadions hat dieses Produkt in seiner reinen Form weder in der Humanmedizin noch in der Tierernährung große Bedeutung erlangt. Die Gründe dafür liegen in der Unlöslichkeit des Menadions in Wasser, seiner geringen Stabilität gegen Umgebungseinflüsse (Hitze, Licht) sowie der hautreizenden und zu Entzündungen führenden Eigenschaften. Derivate, die eine gleich gute antihämorrhagische Wirkung wie das Menadion besitzen und die die geschilderten Nachteile nicht bzw. weniger stark aufweisen, sind die Additionsverbindungen von Alkali- bzw. Erdalkalihydrosulfiten an Menadion (u.a. DE-AS 10 33 017, FR 996 080, US 2 331 808, GB 771 180). Diese Derivate, z.B. das Additionsprodukt von Natriumhydrogensulfit an Menadion, allgemein als MSB (Menadionesodiumbisulfite) bezeichnet, haben in der Futtermittelindustrie große wirtschaftliche Bedeutung erlangt.

Wissenschaftliche Untersuchungen (vgl. M. Carmack, M.B. Moore und M.E. Balis, J . Amer. Chem. Soc. 72, 844-847 und J.C. Vire, G.J. Patrlavche und G.D. Christian, Analytical Chemistry 51 (1979), S. 752 ff) sowie Erfahrungen in der Futtermittelindustrie (J.Nir.J. Kafvi und R. Cohen, Poultry Science, Vol. 57 (1978), S. 206-209) zeigen, daß selbst MSB als das am meisten eingesetzte Bisulfit-Addukt keine ausreichende Stabilität gegenüber Licht, Hitze und Feuchtigkeit besitzt.

Aus diesem Grunde hat man auch schon versucht, die Stabilität des MSB dadurch zu erhöhen, daß man es durch Reaktion mit geeigneten Salzen von schwachen Basen in schlechter wasserlösliche Produkte überführt.

Von diesen Verbindungen hat das Menadionpyrimidinolbisulfit (MPB) die größte Verbreitung gefunden (vgl. US 3 328 169). Dieses Addukt enthält Menadion in einer Menge von 45,6 %, daneben jedoch auch 32,6 % Dimethylpyrimidinol. Die in der Tat zu beobachtende höhere Stabilität dieser Verbindung wird jedoch durch die Anwesenheit einer körperfremden Substanz und einer beabsichtigten, geringeren Wasserlöslichkeit erkauft.

Wegen der erwünschten Überführung des Menadions in ein gut wasserlösliches Derivat, stellen diese Derivate demnach einen Rückschritt dar.

Nach den Angaben der DE-OS 28 55 851 hat man daher versucht, die stabilisierende Wirkung des körperfremden Dimethylpyrimidinols durch bestimmte Vitamine, wie Vitamin $B_1$ oder $B_5$ (Nikotinsäure bzw. Nikotinamid), zu ersetzen. Diese Vitamin-Addukte sind vom Standpunkt der Physiologie sicher unbedenklich, sie weisen jedoch noch die folgenden Nachteile auf.

Die in der DE-OS 28 55 851 genannten Verbindungen besitzen nicht nur eine antihämorrhagische Wirkung, sondern auch eine ausgeprägte Wirkung des zur Stabilisierung eingesetzten Vitamins (vgl. hierzu DE-OS 28 55 851, Seite 8, Abs. 3). Beide Wirkungen sind unlösbar in diesen Verbindungen vereint. Soll beispielsweise die Additionsverbindung der Menadionsulfonsäure an Nikotinsäure zur Vorbeugung oder zur Heilung von Hämorrhagien appliziert werden, wird zwangsläufig auch die physiologisch ganz anders wirksame Nikotinsäure zugeführt.

Gemäß DE-A-1 202 624 wird ein saurer Zuschlagstoff zur Stabilisierung von MSB in der Futtermittel-Vormischung eingesetzt. Er wird lediglich mechanisch zugemischt und hat daher nur geringen Einfluß auf die Stabilität des MSB.

Der Erfindung lag daher die Aufgabe zugrunde, ein antihämorrhagisches Produkt herzustellen, welches die günstigen Wirkeigenschaften von MSB und nicht die nachteiligen Anwendungseigenschaften der oben beschriebenen stabilisierten Derivate besitzt.

Überraschenderweise wurde nun gefunden, daß man dieses Ziel mit dem Wirkstoff MSB selbst erreichen kann. Dazu ist es lediglich erforderlich, das handelsübliche fein-kristalline MSB mit einer physiologisch verträglichen organischen oder anorganischen Säure zu verbinden und in eine Trockenform zu überführen.

Gemäß der vorliegenden Erfindung ist es möglich, unter Anwendung zweier Varianten hervorragend stabile Formulierungen herzustellen.

A. Gemäß der ersten Variante werden Formulierungen hergestellt, die einen relativ hohen Menadionnatriumbisulfit-Gehalt von 90 bis 99 Gew.% und zusätzlich 1 bis 10 Gew.%, jeweils bezogen auf die Formulierung, einer physiologisch verträglichen organischen oder anorganischen Säure aufweisen.

Diese Formulierungen werden bevorzugt durch Sprühwirbelschicht- oder Sprühgranulationstrockung einer wäßrigen Lösung von Menadionnatriumbisulfit und zusätzlich geringen Mengen einer physiologisch verträglichen organischen oder anorganischen Säure erhalten.

Unter Sprühwirbelschichttrocknung wird dabei verstanden, daß die Lösung oder Suspension kontinuierlich oder diskontinuierlich in eine Wirbelschicht aus Produkt eingesprüht wird. Die Einrichtung ist mit geeigneten Vorrichtungen versehen, die es gestatten, eine bestimmte Partikelgrößenfraktion zu gewinnen und den Granulationsprozeß aufrecht zu erhalten (K. Kröll, Trocknungstechnik, Band II: "Trockner und Trocknungsverfahren", 2. Auflage, Springer Verlag, Berlin 1978). Bei der Sprühgranuliertrocknung wird die Lösung oder Suspension in einen geeigneten Sprühturm eingesprüht und getrocknet,

Die Formulierung einer Lösung von Menadionnatriumbisulfit ohne den Säurezusatz gelingt nicht.

Somit haben die zugesetzten Säuren eine Doppelfunktion. Einmal erhöhen sie die Stabilität der so gewonnenen Formulierungen und außerdem ermöglichen sie erst durch ihre Anwesenheit den Sprühgranulierprozeß.

Als Säuren kommen in erster Linie die im deutschen Futtermittelrecht erwähnten Produkte, wie Fumarsäure, Sorbinsäure, Citronensäure, Milchsäure, Phosphorsäure, primäres Calciumphosphat und besonders Weinsäure in Betracht.

Im Vergleich zu den bekannten stabilisierten Produkten weisen die in dieser Weise erhältlichen erfindungsgemäßen Formulierungen folgende Vorteile aus:

1. höhere Stabilität;
2. nahezu gleicher Menadion-Gehalt wie im Menadionnatriumbisulfit;
3. einfachere Handhabung, da die Produkte staubarm und sehr gut rieselfähig sind;
4, gleichgute Wasserlöslichkeit wie nicht formuliertes Menadionnatriumbisulfit, und damit sehr gute biologische Verfügbarkeit;
5. geringere Herstellkosten als die der vorbekannten Formulierungen;
6. keine Belastung durch aquimolekulare chemische Stabilisierungsprodukte, wie Dimethylpyrimidinol.

B. Gemäß der zweiten Variante der Erfindung werden pulverförmige Menadionnatriumbisulfit-Formulierungen hergestellt, die einen Gehalt von 50 bis 80 Gew.% Menadionnatriumbisulfit, 1 bis 20 Gew.% einer physiologisch verträglichen organischen oder anorganischen Säure und 1 bis 49 Gew.%, jeweils bezogen auf die Formulierung, eines Verdickungsmittels aufweisen.

Diese Formulierungen werden zweckmäßig durch Eintrocknen auf Blechen oder in geeigneten Gefäßen in dünner Schicht oder besonders vorteilhaft durch Versprühen und Trocknen im Wirbelbett einer konzentrierten wäßrigen Lösung oder Suspension von Menadionnatriumbisulfit, einer physiologisch verträglichen organischen oder anorganischen Säure und einem Verdickungs- oder Bindemittel erhalten.

Als Säuren kommen gleichermaßen die unter (A) genannten Säuren in Betracht.

Als Verdickungs- oder Bindemittel sind Kohlehydrate, wie Stärke oder Stärkederivate sowie Dextrine oder Proteine, wie Gelatine, zu nennen. Diese Stoffe erhöhen sowohl die Viskosität in einem Bereich, der für die Versprühung günstig ist und dienen als Kitt, der die Wirkstoffteilchen in der getrockneten Form in größeren Agglomeraten zusammenhält, die durch gute Fließfähigkeit und Staubarmut günstige anwendungstechnische Eigenschaften aufweisen.

Im Vergleich zu den bekannten stabilisierten Produkten weisen die in dieser Weise erhältlichen erfindungsgemäßen Formulierungen folgende Vorteile aus:

1. höhere Stabilität;
2. keine Belastung durch äquimolekulare Stabiliserungsfaktoren, wie Dimethylpyrimidol;
3. bessere Handhabung, da die Produkte nicht stauben.

Variante A

Herstellungsbeispiele

A1. Eine konzentrierte wäßrige Lösung aus 30 Teilen MSB als Bishydrat, 0,6 Teilen Citronensäure und 69,4 Teilen Wasser von 20°C wurde über eine Düse in eine Wirbelschicht aus MSB-Teilchen der mittleren Größe 1 - 4 μm gesprüht. Im Zeitabstand von einer Stunde wurde der Inhalt des Wirbelbetts gesiebt und die Teilchen der Größe 125 - 250 μm (≈ 60 %) des Wirbelbettanteils) isoliert. Das Wirbelbett wurde mit einem Gasstrom von 90°C gespeist, die Austrittstemperatur betrug 50°C.

A2. Unter identischen Sprühbedingungen wurde eine 25°C warme Lösung aus 33,4 Teilen MSB, 1,6 Teilen Fumarsäure und 65 Teilen Wasser in das Wirbelbett eingedüst. Der Anteil an Teilchen der Größe 125 - 250 μm betrug nach Siebung 55 - 56 %.

A3. Eine 35°C warme Lösung aus 40 Teilen MSB, 0,6 Teilen Weinsäure und 58,2 Teilen Wasser wurde wie in Beispiel 1 beschrieben, versprüht. Man erhielt eine Nutzfraktion von 125 - 250 $\mu$m großen Teilchen, die 60 bis 80 % des Inhalts vom Wirbelbett ausmachte.

Lagerstabilität

Die Lagerstabilität der erfindungsgemäßen Formulierung wurde durch einen Streß-Test beurteilt: Ein Prämix der Zusammensetzung 70 % Weizengrießkleie, 20 % Cholinchlorid (50 %ig, Träger $SiO_2$) und 10 % einer Spurenelementemischung und 0,1 % der erfindungsgemäßen Formulierung wurden in einem Klimaschrank bei 70 % rel. Luftfeuchte und 40°C gelagert. Nach 1 Woche wurden Proben entnommen und nach der im USP XX beschriebenen Methode der Anteil des wiederauffindbaren Menadions bestimmt.

Die Produkte aus den Beispielen A1. - A3. wurden dem beschriebenen Stabilitätstest unter Streß-Bedingungen unterworfen.

Man fand folgende Rest-Gehalte an Menadion, in %, Anfangswert 100 %, nach einer Lagerzeit von einer Woche.

| 1. | Produkt Beispiel A 1 | 78,4 |
|----|----------------------|------|
| 2. | Produkt Beispiel A 2 | 69,3 |
| 3. | Produkt Beispiel A 3 | 82,4 |
| 4. | Menadionsulfit-dimethylpyrimidolium-addukt | 49,1 |
| 5. | Menadionsulfit-nikotinsäureamid-addukt | 51,4 |
| 6. | MSB | 22,0 |

A4. Eine Lösung von 0,6 Teilen Weinsäure, 65,4 Teilen $H_2O$ und 34 Teilen MSB wurde kontinuierlich in ein Wirbelbett eingesprüht. Ein Teil der Vorlage wurde ständig ausgetragen und klassiert. Die Nutzfraktion von 125 bis 250 $\mu$m wurde isoliert, der gemahlene Grob- mit dem Feinanteil und überschüssige Nutzfraktion in das Wirbelbett rückgeführt. Das Wirbelgas strömte mit 85 bis 95°C ein und mit 48 bis 55°C aus.

Variante 8

Herstellungsbeispiele

B1. 100 Teile Menadionnatriumbisulfit, 20 Teile Maisstärke und 40 Teile Gelatine wurden mit 20 Teilen primärem Calciumphosphat in 600 Teilen $H_2O$ warm gelöst und in einer 1 cm dicken Schicht im Vakuumtrockenschrank bei 400°C und 25 mbar Druck eingetrocknet. Das Produkt wurde anschließend zerstoßen und wie unten beschrieben die Stabilität bestimmt.

B2. Eine warme Lösung aus 100 Teilen Menadionnatriumbisulfit, 25 Teilen Citronensäure und 75 Teilen quellbarer Maisstärke wurden, wie in Beispiel 1 angegeben, eingetrocknet. Man erhielt nach Zerstoßen ein weißes Pulver mit 6,4 % Restfeuchte und einem nach USP XX, S. 473 bestimmten Menadionanteil von 25,1 %.

B3. Eine 50°C warme Suspension aus 100 Teilen Menadionnatriumbisulfit, 20 Teilen Weinsäure und 60 Teilen quellfähiger Stärke in 150 Teilen Wasser wurde in Gegenwart hydrophober Kieselsäure über eine Düse zerstäubt und anschließend im Wirbelbett bei 28 bis 38°C getrocknet. Man erhielt ein ausgezeichnet fließfähiges Trockenpulver mit einem nach USP XX bestimmbaren Menadionanteil von 29,5 %.

B4. Eine 70°C warme Suspension aus 100 Teilen Menadionnatriumbisulfit als Bishydrat, 21,4 Teilen Sorbinsäure und 57,1 Teilen quellfähiger Stärke in 150 Teilen Wasser wurde, wie in Beispiel B3. angegeben, verdüst und mit 3,8 Teilen hydrophobem Kieselgel bepudert. Das Trockenpulver enthielt 6,5 % wäßrige Restfeuchte und einen Anteil von 30,4 % abspaltbarem Menadion.

Lagerstabilität

Die Lagerstabilität des gemäß Variante 8 formulierten Menadionnatriumbisulfits wurde durch einen Streß-Test beurteilt. Ein Prämix der Zusammensetzung 70 % Weizengrießkleie, 20 % Cholinchlorid (50 %ig, Träger $SiO_2$), 10 % einer Spurenelementmischung und 0,1 % des formulierten Menadionnatriumbisulfits - bezogen auf den abspaltbaren Menadiongehalt - wurden in einem Klimaschrank bei 70 % relativer Feuchte und 40° C gelagert. Nach einer Woche wurde der Anteil des wiederauffindbaren Menadions nach der in USP XX S. 473 beschriebenen Methode bestimmt.

Stabilitäten der Trockenpulver im Vergleich zu den stabilisierten Produkten Menadionsulfit-dimethylpyrimidoliumaddukt, Menadionsulfit-nikotinsäureamidaddukt und handelsüblichem Menadionnatriumbisulfit ohne Stabilisator nach 1 Woche:

| Produkt nach Beispiel | % wiederauffindbares Menadion |
|---|---|
| 1 | 76 |
| 2 | 81 |
| 3 | 97 |
| 4 | 92 |
| Menadionsulfit-dimethylpyrimidolium-addukt | 46 |
| Menadionsulfit-nikotinsäureamidaddukt | 45 |
| Menadionnatriumbisulfit, Handelsware MSB (nicht stabilisiert) | 21 |

**Patentansprüche**

1. Verfahren zur Herstellung einer pulverförmigen oder granulierten Menadionnatriumbisulfit-Formulierung durch Sprühgranulierung einer Konzentrierten wäßrigen Lösung von 90 bis 99 Gew.-% Menadionnatriumbisulfit und 1 bis 10 Gew.-%, jeweils bezogen auf die Formulierung, einer physiologisch verträglichen organischen oder anorganischen Säure.

2. Verfahren zur Herstellung einer pulverförmigen oder granulierten Menadionnatriumbisulfit-Formulierung durch Eintrocknen oder Versprühen und Trocknen im Wirbelbett einer konzentrierten wäßrigen Lösung oder Suspension von 50 bis 80 Gew.-% Menadionnatriumbisulfit, 1 bis 20 Gew.-% einer physiologisch verträglichen organischen oder anorganischen Säure und 1 bis 49 Gew.-%, jeweils bezogen auf die Formulierung, eines Verdickungsmittels.

3. Verfahren zur Herstellung einer pulverförmigen oder granulierten Menadionnatriumbisulfit-Formulierung gemäß Anspruch 1, dadurch gekennzeichnet, daß die Säure ausgewählt wird aus Fumarsäure, Sorbinsäure, Citronensäure, Milchsäure, Phosphorsäure, Weinsäure und primärem Calciumphosphat.

4. Verfahren zur Herstellung einer pulverförmigen oder granulierten Menadionnatriumbisulfit-Formulierung gemäß Anspruch 2, dadurch gekennzeichnet, daß als Verdickungs- oder Bindemittel ein Kohlehydrat und/oder ein Protein gewählt wird.

5. Verfahren zur Herstellung einer pulverförmigen oder granulierten Menadionnatriumbisulfit-Formulierung gemäß Anspruch 4, dadurch gekennzeichnet, daß als Verdickungsmittel Stärke oder Gelatine gewählt wird.

6. Verfahren zur Herstellung einer pulverförmigen oder granulierten Menadionnatriumbisulfit-Formulierung gemäß Anspruch 1, dadurch gekennzeichnet, daß als Säure Weinsäure eingesetzt wird.

**Claims**

1. A process for the preparation of a powdered or granulated menadione sodium bisulfite formulation by spray granulation of an aqueous solution of from 90 to 99% by weight of menadione sodium bisulfite and from 1 to 10% by weight of a physiologically tolerated organic or inorganic acid, the percentages in each case being based on the formulation.

2. A process for the preparation of a powdered or granulated menadione sodium bisulfite formulation by drying, or spraying and drying, in a fluidized bed, a concentrated aqueous solution or suspension of from 50 to 80% by weight of menadione sodium bisulfite, from 1 to 20% by weight of a physiologically tolerated organic or inorganic acid and from 1 to 49% by weight of a thickener, the percentages in each case being based on the formulation.

3. A process for the preparation of a powdered or granulated menadione sodium bisulfite formulation as claimed in claim 1, wherein the acid is selected from the group consisting of fumaric acid, sorbic acid, citric acid, lactic acid, phosphoric acid, tartaric acid and primary calcium phosphate.

4. A process for the preparation of a powdered or granulated menadione sodium bisulfite formulation as claimed in claim 2, wherein the thickener or binder is a carbohydrate or a protein.

5. A process for the preparation of a powdered or granulated menadione sodium bisulfite formulation as claimed in claim 4, wherein the thickener is starch or gelatine.

6. A process for the preparation of a powdered or granulated menadione sodium bisulfite formulation as claimed in claim 1, wherein the acid is tartaric acid.

## Revendications

1. Procédé de préparation d'une formule de sulfite de sodium ménadione pulvérulente ou granulée par granulation en fines gouttelettes d'une solution aqueuse de 90 à 99 % en poids de bisulfite de sodium ménadione et de 10 % en poids, rapportés chacun à la formulation, d'un acide organique ou inorganique acceptable physiologiquement.

2. Procédé de préparation d'une formule de sulfite de sodium ménadione pulvérulente ou granulée par séchage ou pulvérisation et séchage en couche en mouvement turbulent d'une solution aqueuse concentrée ou suspension de 50 à 80 % en poids de bisulfite de sodium-ménadione, 1 à 20 % en poids d'un acide organique ou inorganique acceptable physiologiquement et 1 à 49 % en poids, rapportés chacun à la formulation, d'un agent d'épaississement.

3. Procédé de préparation d'une formule de sulfite de sodium ménadione pulvérulente ou granulée selon la revendication 1, caractérisé par le fait que l'acide est choisi parmi les acides fumarique, sorbique, citrique, lactique, phosphorique, tartrique et le phosphate de calcium primaire.

4. Procédé de préparation d'une formule de sulfite de sodium-ménadione pulvérulente ou granulée selon la revendication 2, caractérisé par le fait que l'on choisit, comme agent d'épaississement ou de liant, un hydrate de carbone et/ou une protéine.

5. Procédé de préparation d'une formule de sulfite de sodium-ménadione pulvérulente ou granulée selon la revendication 4, caractérisé par le fait que l'on choisit, comme agent d'épaississement, de l'amidon ou de la gélatine.

6. Procédé de préparation d'une formule de sulfite de sodium ménadione pulvérulente ou granulée selon la revendication 1, caractérisé par le fait que, comme acide, on utilise de l'acide tartrique.